# EUROPEAN PATENT APPLICATION

(11) **EP 4 679 448 A1**
(43) Date of publication of application: **14.01.2026**
(21) Application number: 24186995.7
(22) Date of filing: 08.07.2024
(51) Int. Cl.: G16H 40/40, A61B 5/00

(54) **SENSOR OPTIMIZATION SYSTEM**

(71) Applicant: Koninklijke Philips N.V., 5656 AG Eindhoven (NL)
(72) Inventor: VISSER, Jeffrey, Eindhoven (NL); VAN LIESHOUT, Ron Martinus Laurentius, Eindhoven (NL); BINGLEY, Peter, 5656AG Eindhoven (NL); HUIJBREGTS, Laurentia Johanna, Eindhoven (NL)
(74) Representative: Philips Intellectual Property & Standards

(57) **Abstract**

The disclosed system and method manage computational resources in a monitoring system that receives medical data from multiple sensors. A suitability score is obtained for each sensor, which may indicate the accuracy and/or volume of the medical data it generates, an energy consumption efficiency of the sensor, and/or a delay associated with generation of physiological parameter values from medical data generated by the sensor. The system identifies duplicate sensors, each capable of generating data for deriving the same physiological parameter. The suitability scores of these duplicate sensors are processed to identify a redundant sensor. The system then modifies the operating state of the redundant sensor or alters the processing operation applied to the data it generates. This approach balances computational resource usage with clinical requirements, optimizing the performance of the monitoring system.

## Description

### FIELD OF INVENTION

The present invention relates to monitoring systems, and more specifically, to methods and systems for optimizing sensor control to reduce computational load on the monitoring system.

### BACKGROUND

Monitoring systems are a cornerstone of modern healthcare, providing real-time data on patient physiological parameters. These systems typically rely on a multitude of sensors, each designed to capture medical data from which physiological parameters such as heart rate, blood pressure, or respiratory rate can be derived. As technology advances, the number of sensors available, and the number of parameters that can be monitored, is continually increasing. This leads to a wealth of data that can be used to inform patient care.

However, this increase in data comes with its own challenges. Each sensor requires computational resources to process the data it captures, so as to generate the corresponding physiological parameter values, as well as transfer, process, present, and store the data and/or resultant parameter values. Thus, as the number of sensors increases, so does the computational load on the processing unit of the monitoring system. This can limit the system's ability to perform real-time calculations, particularly in integrated platforms that support a finite amount of these calculations.

Furthermore, the increasing number of sensors can lead to redundancy in the data. For instance, multiple sensors may be capable of capturing data that may be used to derive the same physiological parameter, resulting in duplicate measurements. This redundancy not only increases the computational load on the system, but also can lead to unnecessary data storage and transfer, further straining the system's resources.

Therefore, there exists a need for a system that may more effectively balance clinical requirements and the increased volume of data.

### SUMMARY OF INVENTION

The invention is defined by the claims.

According to an aspect of the present disclosure, a method is provided for controlling a monitoring system configured to receive medical data of a subject from a plurality of sensors, and to process the medical data to generate physiological parameter values of the subject.

The method includes obtaining, for each of the plurality of sensors, a suitability score of the respective sensor. The method further includes processing the obtained suitability scores associated with each of a plurality of duplicate sensors amongst the plurality of sensors to identify a redundant sensor. Each of the duplicate sensors are configured to generate medical data suitable for deriving a value of a same physiological parameter. The method also includes controlling the monitoring system to modify an operating state of the redundant sensor and/or to modify a processing operation applied by the monitoring system to the medical data generated by the redundant sensor. This method allows for efficient use of computational resources by identifying and managing redundant sensors, thereby optimizing the performance of the monitoring system.

The present disclosure introduces concepts for optimizing data source selection for monitoring systems, thereby reducing the computational load on the system's processing unit. Embodiments may leverage the concept of suitability scoring, where each sensor is assessed and assigned a score. For example, the data accuracy of each sensor (in a set of sensors that generate data that may be used to generate a same or similar physiological parameter) may be assessed and assigned a score (such as a confidence score). Alternatively, or in addition, various other aspects may be considered such as the volume of data generated by the sensor, the energy consumed by the sensor, etc. This score is then used to identify redundant sensors amongst the duplicate sensors, i.e., sensors that generate unnecessary duplicate measurements of the same physiological parameter. By identifying and managing these redundant sensors, the system can optimize its computational resources, leading to improved performance and efficiency.

Proposed embodiments may provide several advantages. Firstly, they may allow for efficient use of computational resources by identifying and managing redundant sensors, thereby optimizing the performance (e.g., processing, energy consumption, data load, etc.) of the monitoring system. Secondly, embodiments may provide flexibility in managing the operations of the redundant sensors and the processing of their generated data, thereby further optimizing the computational resources of the monitoring system. Thirdly, embodiments may adapt to different clinical, system, and user requirements, ensuring that the monitoring system provides the desired physiological parameter values. Lastly, embodiments may allow for continuous monitoring and management of the sensors, ensuring that the monitoring system operates efficiently and provides accurate physiological parameter values.

Put another way, it has been realized that monitoring systems often receive multiple sets of medical data that may be used to generate a same physiological parameter. Accordingly, unnecessary measurements by sensors and processing by the monitoring system, may be performed. That is, a same physiological parameter value may be generated from multiple data streams. In order to resolve this issue, the invention proposes the use of suitability scores describing/indicating suitability of each sensor with respect to clinical, system and/or user requirements. By processing these suitability scores, redundant sensors may be identified.

For example, if the suitability scores indicate that one sensor amongst the duplicate sensors generates data that is significantly more accurate than other sensors amongst the duplicate sensors, then the other sensors may be considered redundant. Alternatively, if the suitability scores indicate that each of the duplicate sensors are considered to be inadequately accurate (i.e., each generate data having a relatively low-level of accuracy), then none or few of the sensors may be considered redundant in order to ensure the generation of a physiological parameter that is accurate. Of course, other scenarios are possible.

Once a redundant sensor is identified, mitigating actions may be taken by the monitoring system. The monitoring system may modify an operating state of the redundant sensor, for example by switching the redundant sensor off, into a low-power state, or another state where data acquisition is reduced. Alternatively, or in addition, the monitoring system may modify the processing operation of the medical data generated by the redundant sensor. This may include, for example, reducing the rate or a complexity of calculations or algorithms applied to the medical data, or altering a data transfer and storage process.

In some embodiments, the suitability score may indicate/describe at least one of an accuracy of medical data generated by the sensor, an energy consumption efficiency of the sensor, a volume of medical data generated by the sensor, or a delay associated with generation of physiological parameter values from medical data generated by the sensor.

That is, the suitability score may provide means for ranking the sensors based on the accuracy or volume of medical data generated by the sensor, an energy consumed by the sensor in generating the medical data, or a delay associated with the sensor. Indeed, any combination of these factors may be reflected in the suitability score. Of course, these factors only represent an exemplary list of factors, and further factors impacting suitability of the sensors may also be considered.

Which of these factors, and the weighting of these factors, may be determined based on particular user or healthcare provider preferences. Indeed, this enables for the consideration of a wide variety of parameters associated with the sensors, in order to appropriately rank the sensors.

In some embodiments, controlling the monitoring system may include replacing or disabling a measurement operation of the redundant sensor, replacing or disabling a transfer operation for medical data generated by the redundant sensor, and/or replacing or disabling algorithms and calculations performed to generate physiological parameter values using the medical data generated by the redundant sensor. This provides flexibility in managing the operations of the redundant sensor and the processing of its generated medical data, thereby further optimizing the computational resources of the monitoring system.

In various embodiments, when the suitability score comprises a confidence score indicating an accuracy of medical data generated by the sensor, obtaining the suitability score of each sensor may include obtaining sensor context data comprising expected sensor quality information, historical sensor quality information, sensor placement information, sensor use information, sensor interference information, and/or perceived sensor quality information. Then, the confidence score may be determined based on the sensor context data. This enables accurate generation of the confidence score taking into account various factors associated with the respective sensor.

In additional or alternative embodiments, the suitability score comprises a confidence score indicating an accuracy of medical data generated by the sensor, obtaining the suitability score of each sensor comprises processing, for each of the plurality of sensors, medical data obtained from the respective sensor to determine the confidence score. This enables accurate generation of the confidence score taking into account the medical data received from the sensor. For example, the confidence score may relate to the signal-to-noise ratio of the medical data wherein the medical data is in the form of a raw signal, or changes in the physiological parameter value that cannot be physiologically correct.

In further embodiments, the method may include obtaining a clinical, system and/or user requirement indicating a target characteristic of medical data relating to a physiological parameter, and identifying the redundant sensor of the plurality of sensors further based on the obtained clinical, system and/or user requirement. This allows for the method to adapt to different clinical, system, and user requirements, thereby ensuring that the monitoring system provides the desired physiological parameter values. Accordingly, minimum requirements may not be sacrificed by modifying control of an identified redundant sensor.

Furthermore, embodiments may include a further step of processing the obtained suitability scores associated with each duplicate sensor of the plurality of sensors to identify a further redundant sensor. Indeed, there may be multiple redundant sensors in the set of duplicate sensors. It may therefore be the case that medical data from more than one of the duplicate sensors is redundant. Accordingly, this means that the processing capacity required by the monitoring system may be further reduced.

Embodiments of the invention may also include an additional step of identifying the duplicate sensors amongst the plurality of sensors. That is, the invention may not obtain a list describing duplicate sensors amongst the plurality of sensors, but the duplicate sensors may be identified. This may improve flexibility of the method, enabling the categorization of previously unknown sensors.

Specifically, identifying the duplicate sensors may include obtaining, for each of the plurality of sensors, a list of physiological parameters derivable from medical data generated by the respective sensor. The list of physiological parameters of each of the plurality of sensors may then be compared to identify one or more sets of duplicate sensors, each set of duplicate sensors comprising sensors configured to generate medical data suitable for deriving a value of a same physiological parameter. This means that the duplicate sensors may be reliably identified.

Alternatively, or additionally, identifying the duplicate sensors may include comparing, for each pair of sensors of the plurality of sensors, medical data generated by each sensor, and identifying duplicate sensors based on a result of the comparison. This therefore provides that the duplicate sensors may be identified by the medical data itself. Accordingly there is no need to obtain additional information regarding the sensors (e.g., the list described above), but requires additional processing.

In yet other embodiments, the method may include repeating the steps of obtaining the suitability scores, processing the obtained suitability scores, and controlling the monitoring system according to a predefined loop function. This allows for continuous monitoring and management of the sensors, thereby ensuring that the monitoring system operates efficiently and provides accurate physiological parameter values.

More particularly, the steps of obtaining the suitability scores, processing the obtained suitability scores, and controlling the monitoring system may be repeated responsive to an external trigger. The external trigger may comprise, for example, the monitoring system receiving medical data from a new sensor, a user intervention, or a change to a clinical, system and/or user requirement. This avoids unnecessary repetition of steps, only repeating when there is a need to update management of the sensors, thereby ensuring efficiency.

Additionally, or alternatively, the steps of obtaining the suitability scores, processing the obtained suitability scores, and controlling the monitoring system are repeated responsive to a predetermined amount of time having elapsed. Thus, it may be ensured that the management of the sensors is updated in a predictable manner, thereby ensuring that the monitoring system operates efficiently and provides accurate physiological parameter values.

The predetermined amount of time may be based on the same physiological parameter. That is, the predetermined amount of time may depend on the physiological parameter derivable from the duplicate sensors. Thus, the management of the sensors is updated in an appropriate manner taking into account the physiological parameter measured by the sensors.

According to another aspect of the present disclosure, a control system is provided for a monitoring system configured to receive medical data of a subject from a plurality of sensors, and to process the medical data to generate physiological parameter values of the subject. The control system includes an assessment module configured to obtain, for each of the plurality of sensors, a suitability score of the respective sensor. The control system also includes a redundant check module configured to process the obtained suitability scores associated with each of a plurality of duplicate sensors amongst the plurality of sensors to identify a redundant sensor. Each of the duplicate sensors are configured to generate medical data suitable for deriving a value of a same physiological parameter. The control system further includes a communication module configured to control the monitoring system to modify an operating state of the redundant sensor and/or to modify a processing operation applied by the monitoring system to the medical data generated by the redundant sensor. This control system provides an efficient way to manage the sensors and the processing of their generated medical data, thereby optimizing the performance of the monitoring system.

Put another way the disclosed system may comprise three main modules: the assessment module, the redundancy check module, and the communication module. The assessment module assesses the suitability of each sensor, and may take into account factors such as expected, historical and perceived sensor measurement quality, sensor measurement volume, sensor energy efficiency, and sensor delay. The redundancy check module then processes the suitability scores of duplicate sensors (i.e., sensors that generate data from which a same physiological parameter may be derived) to identify which ones are redundant and ones whose control and associated processes can be modified. Finally, the communication module interacts with the main system to modify the operations of the redundant sensors and the processing of their generated data.

In some embodiments, the system may further comprise a duplication check module configured to identify the duplicate sensors amongst the plurality of sensors. The duplication check module identifies duplicate sensors, i.e., sensors that may be used to obtain the same physiological parameter.

These and other aspects of the invention will be apparent from and elucidated with reference to the embodiment(s) described hereinafter.

### BRIEF DESCRIPTION OF FIGURES

For a better understanding of the invention, and to show more clearly how it may be carried into effect, reference will now be made, by way of example only, to the accompanying drawings, in which:
FIG. 1 is a flowchart illustrating a method for controlling a monitoring system, according to aspects of the present disclosure.
FIG. 2 is a block diagram depicting a control system for a monitoring system, according to aspects of the present disclosure.
FIG. 3 is a block diagram showing a control system within a system housing, according to aspects of the present disclosure.

### DETAILED DESCRIPTION

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

The present disclosure relates to a system and method for managing data processing in monitoring systems. In particular, the present disclosure may provide a system and method for optimizing the use of computational resources in monitoring systems by identifying and managing redundant data sources. This approach may help to balance the computational resources and clinical requirements of the system, potentially improving the efficiency of patient monitoring and reducing energy consumption.

More specifically, disclosed concepts manage computational resources in a monitoring system that receives medical data from multiple sensors. A suitability score is obtained for each sensor, which may reflect/indicate the accuracy, quality and/or volume of the medical data it generates, the energy/power the sensor consumes, and/or a delay associated with data acquisition by the sensor. The system identifies duplicate sensors, each capable of generating data for deriving the same physiological parameter. The suitability scores of these duplicate sensors are processed to identify a redundant sensor. The system then modifies the operating state of the redundant sensor or alters the processing operation applied to the data it generates. This approach balances computational resource usage with clinical requirements, optimizing the performance of the patient monitoring system.

When embodied as a system, there may be provided an assessment module, a duplicate check module, a redundancy check module, and a communication module. The assessment module may assess the accuracy, volume and quality of measurements from various data sources, such as patient monitors, ventilators, wearable monitor devices, and others, the energy consumption by the various data sources, and a delay associated with the data sources. That is, the assessment module determines the suitability of various data sources with respect to various requirements (e.g., accuracy/quality requirements, energy consumption requirements, delay requirements, etc.). The duplicate check module may identify duplicate sensors, i.e., sensors capturing medical data that can be used to derive the same physiological parameter. The redundancy check module may determine whether any of the duplicate measurements are redundant and can be made redundant without compromising the clinical or system requirements. The communication module may interact with the main system to modify the processing of measurements, algorithms, and calculations based on the findings of the redundancy check module.

Furthermore, embodiments may repeat the process of assessment, duplicate check, redundancy check, and communication to ensure the continuous optimization of computational resources. This loop function may operate at different frequencies depending on various factors, such as the addition of new measurements, changes in clinical or system requirements, or human intervention.

The method of the present disclosure may involve obtaining suitability scores for each data source, identifying duplicate measurements, determining redundant measurements, and controlling the processing of redundant measurements. This method may help to optimize the use of computational resources in patient monitoring systems, potentially improving the efficiency of patient monitoring and reducing energy consumption.

In some cases, the system and method of the present disclosure may provide a more efficient and effective way to manage data processing in patient monitoring systems. By identifying and managing redundant data sources, the system and method may help to balance the computational resources and clinical requirements of the system, potentially improving the efficiency of patient monitoring and reducing energy consumption.

By way of brief explanation, patient monitor solutions are getting more complex over time. More and more data sources are being integrated into a single monitoring platform. As a result, some physiological parameters are duplicated or derived as secondary measurement from separate primary measurements. Selecting the least computationally expensive source could favor the device in sparing resources. Nevertheless, it is necessary to balance reduction of computational expense with clinical need to process the signals generated from the sensors.

In clinical practice, healthcare professionals are primarily interested in the physiological measurements themselves, rather than the specific sensors used to obtain them. Typically, when a parameter is derived from medical data from multiple different sensors, all the medical data are processed by the monitoring system. This can lead to an inefficient use of computational resources, which may have a negative impact particularly when the system's resources are limited.

Balancing the computational resources and the clinical requirements is a complex task. On the one hand, the system has to ensure the accuracy and reliability of the physiological measurements. On the other hand, it must manage its computational resources efficiently to avoid overloading the processing unit. This balance becomes even more challenging as the number of sensors and parameters continues to increase.

By way of example, respiratory rate of a subject may be derived based on an ECG signal, as well as from a PPG signal. Further, the subject may be mechanically ventilated, and thus the respiratory rate may be derived directly from the ventilation parameters. Typically, all data streams are processed by a monitoring system for signal processing, alarm determination, trend calculation, and display on the screen of the respiratory rate. Since the computational resources on a patient monitor are limited, this may mean that other processes to be performed by the monitor are needlessly sacrificed. Of course, this does not only occur for respiratory rate, but various other physiological parameters such as heart rate and blood pressure, which may be derived from data from various types of sensors.

Accordingly, the invention is based on the need to reduce computational load of the monitoring system whilst still ensuring that clinical needs are satisfied. This is achieved by identification of redundant sensors amongst sets of duplicate sensors (i.e., sensors that generate information that may be used to generate values the same physiological parameter) based on suitability scores reflecting suitability of the sensors for gathering medical data according to user, system and/or clinical requirements. The sensors may then be appropriately managed by the monitoring system. For example, settings of the redundant sensors themselves may be modified (e.g., switched off or switched to a low power mode). Alternatively or additionally, processing of data from the redundant sensors may be modified (e.g., calculations or transfer of data from the redundant sensor may be suspended).

Duplicate sensors may be identified based comparisons of lists of physiological parameters that are derivable from data produced by the sensors. Alternatively, the duplicate sensors may be determined based on the medical data obtained from each sensor. That is, duplication detection may be achieved by comparing signals from sensors. If the signals have an overlap, this may indicate that the sensors are duplicates. This may be achieved based on a raw-signal using fingerprinting methods, or on parameters resulting from the raw-signal by direct comparison.

It is worth noting that the derivation of some physiological parameters requires multiple data streams, and therefore multiple sensors. For example, an ECG measurement may be achieved by various numbers of lead configurations (e.g. V4R, right sided ECG, Lewis-lead, 3-lead, 5-lead, 12-lead ECG, etc.). However, for simpler physiological parameters, e.g., heart rate, fewer leads are required. Therefore, the sensors may be considered duplicates for a heart rate measurement, but non-duplicates for the ECG measurement. Thus, various physiological parameters may be considered when identifying duplicate sensors.

Once the duplicate sensor(s) have been identified, the monitoring system may then determine which duplicate sensor(s) may be considered redundant. This may be primarily performed based on processing confidence scores indicating an expected accuracy of medical data acquired by each sensor, included in the suitability score of the respective sensor. That is, an important aspect of the assessment of the sensors is the accuracy (whether actual, historical, or perceived) of medical data acquired from each sensor.

Nevertheless, the identification of redundant sensors may be further based on other user needs and preferences. Different user needs may include, for example:
(i) Energy saving. For example, in the case of power failure or transport, the monitoring system may rely on a battery. In this case the identification of the redundant sensor may be focused on the sensor that is using a greatest amount of energy. That is, the identification of the redundant sensor may be performed with the aim of reducing the power consumed by the sensors to generate the same physiological sensor;
(ii) Full information. Hospitals often run clinical trials (for research or educational purposes) that need all available data of the patients. In this case, it may be undesirable to identify redundant sensors at all, in order to ensure a maximum amount of medical data is acquired. Furthermore, full information may be needed to fulfill regulatory needs; and
(iii) Data storage and communication saving. The number and the selection of the identified redundant sensor(s) may be based on capacity of data communication (outside the system) and/or data buffer capacity of the monitoring system. Thus, the sensor not identified as redundant may be the sensor which generates the lowest amount of medical data in bytes per second.

Of course, other user, clinical and system requirements may be prescribed, based on which the redundant sensor(s) may be identified.

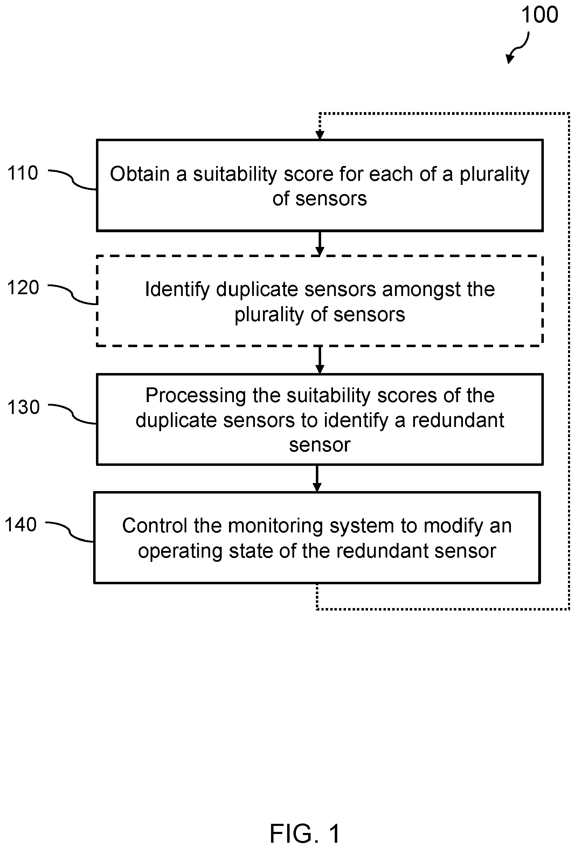

Referring to FIG. 1, a flowchart of a monitoring system control method 100 is depicted. The monitoring system is configured to receive medical data of a subject from a plurality of sensors, and to process the medical data to generate physiological parameter values of the subject.

By way of brief explanation, the received medical data may include a wide variety of data from different sources. The medical data may refer to data from sensors, which may include a raw data signal, a partly processed signal, or a fully processed signal giving values of a physiological parameter. Furthermore, the medical data may also include data from other medical devices (i.e., devices that aren't sensors), which may include device settings, device driving information, etc. For example, this may encompass driving data from a ventilator, from which a respiration rate of the subject can be derived. In any case, all of the medical data may be used to derive one or more physiological parameters of the subject.

The method 100 may start with suitability score acquisition 110, where a suitability score for each sensor in a plurality of sensors (each configured to capture medical data) is obtained.

The suitability score may indicate the accuracy of the medical data generated by each sensor. That is, the suitability score may reflect the quality of the medical data generated by the respective sensor. Alternatively, the suitability score may reflect a volume of medical data acquired, an energy consumption efficiency of the sensor, or a delay associated with generation of physiological parameter values from medical data generated by the sensor. Of course, other factors may also be indicated by the suitability score. In any case, the suitability score provides a means for ranking each of the plurality sensors based on how preferable it would be to obtain medical data from one sensor or the other.

In a simple embodiment, each sensor may be associated with a prescribed suitability score, for example as given by a user. Indeed, if the suitability score is based on the accuracy of medical data generated by the sensor, then this may be derived from parameters provided by a manufacturer (in the form of prescribed accuracy information).

In other cases, the suitability score of each sensor may be determined based on various factors such as the expected quality of the sensor, historical sensor quality information, sensor placement information, sensor use information, sensor interference information, perceived sensor quality information, historical or measured sensor energy consumption, historical or measured medical data volume information, etc.

Put another way, in step 110, the suitability of each sensor with respect to user, clinical and/or system requirements is indicated by the suitability score. Ideally, this step is performed using minimal computational power. Accordingly, the suitability score may be obtained using a look-up table, or based on historical information related to said sensors.

In some cases, the suitability score may comprise a confidence score indicating an accuracy of medical data generated by the respective sensor. In this case, the confidence score may also be determined based, at least in part, on the medical data generated by the sensor itself. That is, the medical data obtained from the sensor may be processed to obtain a quality grading of the measurement. This may typically be performed on the raw signal from the sensor. Accordingly, the confidence score may describe the quality/accuracy of the data related to the placement, use and/or interference linked to the sensor. The particular processing applied to the medical data to determine the accuracy and/or quality will depend on the type of medical data itself (i.e., will depend on the measurement being performed), with quality assessment processing being apparent to the skilled person. Generally, the signal-to-noise ratio may be identified, but processing is not restricted hereto.

Following the suitability score acquisition 110, the method 100 may proceed to (optional) duplicate sensor identification 120. This step may involve identifying duplicate sensors among the plurality of sensors. Duplicate sensors may be defined as sensors that are configured to generate medical data suitable for deriving a value of the same physiological parameter. For example, if two sensors are both capable of generating medical data that can be used to determine/derive heart rate, these sensors may be considered duplicate sensors.

In other words, in step 120 it may be determined which sensors produce medical data that are interchangeable for deriving a value of a physiological parameter. In a straightforward implementation, this may be achieved by use of a look-up table, which indicates what medical data (and thus sensors) are duplicates. Equally, the similarity of the medical data produced by the sensors, and/or the parameter values that are derivable from the medical data may be assessed (both from a numeric point of view and from a historical pattern point of view).

It is worth noting that the definition of which sensors are duplicates is considered on the physiological parameter level. For example, a respiratory rate could be measured using a deviation of the ECG measurement, a deviation of flow measured from a capnography, or using accelerometers. In the clinical context, these sources of medical data have the same value (i.e., can be used to derive the same physiological parameter).

Of course, in some embodiments of the invention, sets of duplicate sensors may already be known, and therefore identification of duplicate sensors is not necessary.

After the duplicate sensor identification 120, the method 100 may proceed to a redundant sensor determination 130. This step may involve processing the obtained suitability scores of the duplicate sensors to identify a redundant sensor.

Generally, the redundant sensor determination 130 may be based on the suitability scores obtained in the suitability score acquisition 110 and the identification of duplicate sensors in the duplicate sensor identification 120.

A redundant sensor may be defined as a sensor that generates medical data that is not necessary for deriving a value of a physiological parameter when considering medical data generated by other sensors, for example because another sensor (or sensors) provides the same or similar data with a higher suitability score. Accordingly, step 130 may simply comprise ranking duplicate sensors based on the suitability score, and identifying a redundant sensor (or sensors) being a sensor associated with the lowest suitability score.

Nevertheless, the identification of the redundant sensor may be more complex than simply identifying a duplicate sensor with the lowest suitability score. For example, if it is determined that none of the duplicate sensors have a high enough suitability score (i.e., none of the suitability scores meet a threshold), then none (or few) of the duplicate sensors may be identified as redundant. In other words, redundant sensors may be identified whilst still ensuring that medical data of a high enough accuracy and quality is being generated by the non-redundant duplicate sensor(s).

Furthermore, step 130 may further comprise obtaining a clinical, system and/or user requirement indicating a target characteristic of medical data relating to a physiological parameter. Identifying the redundant sensor of the plurality of sensors may then be further based on the obtained clinical, system and/or user requirement.

Thus, the non-redundant sensors must generate medical data that satisfies clinical requirements (e.g., there may be the need for equivalent medical data from two sources, or for derivation of other physiological parameters), system requirements (e.g., medical data from a specific sensor may be required to ensure operation of other devices), and user requirements (e.g., the user may simply prefer medical data from particular sensors). Generally, the clinical, system, and user requirements define a desired minimum level of redundancy. Indeed whilst redundancy results in greater computational requirements, the proposed method may be conscious of other needs/pressures that arise from the clinical context.

By way of specific example, when the physiological parameter is a respiratory rate, we may consider the following situations:
(i) The patient has a SpO2 (finger-clip) sensor, and a multi-lead ECG sensor attached to their body. Both the SpO2 sensor and the ECG sensor are capable of generating medical data from which the respiratory rate may be derived. It has been determined that the ECG sensor has a higher suitability score compared to the PPG sensor for derivation of the respiratory rate measurement (e.g., the ECG sensor provides more accurate information for derivation of the respiratory rate than the PPG sensor). In this example, the patient is stable and not on a ventilator. Therefore, it is determined that the SpO2 sensor is a redundant sensor;
(ii) The patient has a SpO2 finger-clip sensor, a multi-lead ECG sensor, and is on a mechanical ventilator. Because the patient's respiration rate is controlled by the mechanical ventilator, both the ECG and PPG sensor may be considered redundant;
(iii) The patient has a SpO2 finger-clip sensor, a multi-lead ECG sensor, and is on a mechanical ventilator. Nevertheless, there is a clinical requirement to understand the impact of the mechanical ventilator and/or preventing error. Accordingly, one of the PPG or ECG sensors are considered redundant. Further, in order to optimize the computational needs, the measurement frequency of the non-redundant sensor is reduced. The system may then activate a measurement when the ventilator settings are abnormal (i.e. triggering an alarm).

The above cases highlight considerations for a clinical situation. However, other elements could determine whether or not a sensor is redundant. For example, performance and robustness of the sensors based on scientific research and post market surveillance studies may ensure that sensors are not considered redundant. In other situations, it may still be important to obtain information from additional sensors for liability reasons. Furthermore, user-interface settings determining which parameter is presented to the user may provide an indication of which sensors are or are not redundant. Other considerations when identifying redundant sensors would be readily apparent to the skilled person.

Finally, the method 100 may conclude with a monitoring system operation modification 140. This step involves controlling the monitoring system to modify the operating state of the redundant sensor.

The modification may include replacing or disabling a measurement operation of the redundant sensor, replacing or disabling a transfer operation for medical data generated by the redundant sensor, and/or replacing or disabling algorithms and calculations performed to generate physiological parameter values or risk scores using the medical data generated by the redundant sensor.

That is processes linked to the identified redundant sensor(s) may be replaced and/or disabled. These can be processes around the measurements, algorithms, UI-components (such as trends, advanced visualizations), and/or calculations. Accordingly, a computational load induced by processing of medical data from redundant sensors is reduced. The particular processes that are replaced or disabled may depend on clinical, user and/or system requirements, and may be further based on the suitability score associated with the redundant sensor (and other non-redundant duplicate sensors).

Accordingly, the monitoring system operation modification 140 may help to optimize the use of computational resources in the monitoring system, potentially improving the efficiency of patient monitoring and reducing energy consumption.

Steps 110-140 may be repeated in order to ensure that system settings are appropriately updated. This may be particularly beneficial as a fail-safe, with the operation of sensors (and thus the accuracy and quality of medical data generated by the sensors) potentially varying with time.

The rate at which these steps are repeated may be based on a predefined loop function that ensures appropriate repetition of these steps. The predefined loop function may cause one or more of the steps to be repeated responsive to an external trigger, for example. The external trigger may be when the monitoring system receives medical data from a new sensor (i.e., another sensor generating medical data that may also be sued to derive the physiological parameter), a user intervention (i.e., a user request to repeat the steps, or an intervention performed on the subject), a change to a clinical, system and/or user requirement, or a sensor alarm (e.g., an INOP alarm that indicates the sensor is performing incorrectly).

In addition (or instead of) the steps being repeated responsive to an external trigger, the steps may be repeated at a regular interval. That is, the steps may be repeated once a predetermined amount of time has elapsed. This amount of time may depend on the physiological parameter derivable from the medical data. For example, sensors suitable for deriving a physiological parameter that is liable to change rapidly may have steps repeated at a faster rate than those suitable for deriving a physiological parameter that changes slowly.

It should also be noted that the steps 110-140 may be performed in a different order to the order depicted in FIG. 1. For example, duplicate sensors may first be identified (step 120) before suitability scores are obtained (step 110). In addition, it should be noted that the duplicate sensors may not be identified (step 120) during every loop of the method 100, nor suitability scores obtained or completely refreshed for every loop. Various orders and combinations of the steps of the method 100 would be readily appreciated and understood by the skilled person.

Accordingly, the method 100 of FIG. 1, enables for continuous appropriate control of the plurality of sensors, thus ensuring a robust system that consumes an appropriate amount of computational power.

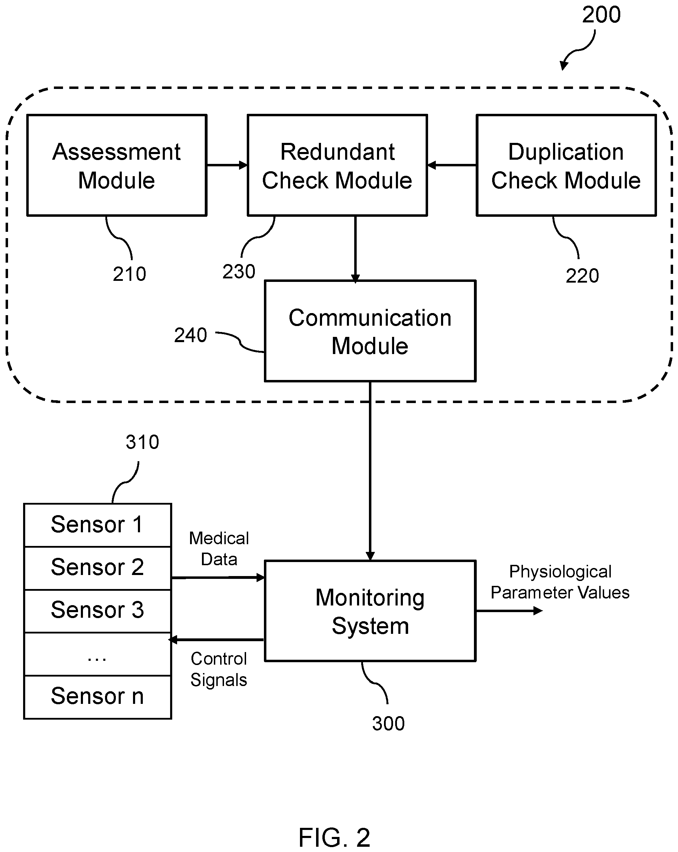

Referring to FIG. 2, a Control System Framework 200 is depicted for optimizing data processing in a Patient Monitoring System 300. The Control System Framework 200 may include an Assessment Module 210, a Duplication Check Module 220, a Redundancy Check Module 230, and a Communication Module 240. These modules may interact with each other and with the Monitoring System 300 to manage the data flow and processing from Medical Data Sensors 310 to the Monitoring System 300, thereby ensuring efficient use of computational resources.

In some aspects, the Assessment Module 210 may evaluate the suitability of various sensors, such as by assessing the accuracy of medical data from the various sensors, or power consumed by the various sensors. The evaluation may be performed at different levels, including expected sensor quality, raw signal quality, and perceived quality of the fully processed signal. For example, the expected sensor quality may be based on a look-up table or historical information related to the quality of the sensors. The raw signal quality may be evaluated based on the signal quality measurements reported by the sensor manufacturer, while the perceived quality may be related to the usage of the measurement, such as the preference for invasive blood pressure measurement over non-invasive blood pressure measurement.

The Duplication Check Module 220 may identify duplicate data sources. In some cases, this identification may be based on a look-up table that indicates which parameters and thus sensors are duplications. In other cases, the Duplication Check Module 220 may compare the similarity of parameters from a numeric and historical pattern perspective to identify duplicate sensors.

The Redundancy Check Module 230 may determine which data sources are redundant and can be disabled or replaced. This determination may be based on the quality assessment results from the Quality Assessment Module 210 and clinical or system requirements. The Redundancy Check Module 230 may consider different clinical situations and other elements such as performance, robustness, data transfer to EMR, and user-interface settings. For example, in a clinical situation where a patient is stable and not on a ventilator, the Redundancy Check Module 230 may determine that a respiration rate parameter calculation based on a SpO2 sensor can be made redundant.

The Communication Module 240 may interact with the Monitoring System 300 to modify operations based on the redundancy assessment. This modification may include replacing or disabling processes around measurements, algorithms, and calculations. The Communication Module 240 may also read the state of the main system regarding sensor inputs, running algorithms, and calculations.

In some cases, the Control System Framework 200 may include a loop function to facilitate a failsafe by repeating the process of quality assessment, duplication check, and redundancy check. The rate of occurrence of this loop function can be high, medium, or low (or may occur based on one-time trigger events) based on different triggers such as the addition of new measurements, changes in clinical or system requirements, or human intervention.

In some aspects, the system can operate in different user modes such as energy saving, full information, data storage & communication saving, and max quality mode, which can modify the behavior of the Redundancy Check Module 230. For example, in the energy saving mode, the Redundancy Check Module 230 may focus on the parameter that is using the lowest amount of processing energy.

In some cases, the system can handle parameters that require multiple sensors and data streams, and can adjust the redundancy check based on the user's requirements. For example, an ECG measurement can be done in various ways with various amounts of leads, and the redundancy check can be adjusted based on the user's requirements for more advanced cardiac measurements versus heart-rate measurements.

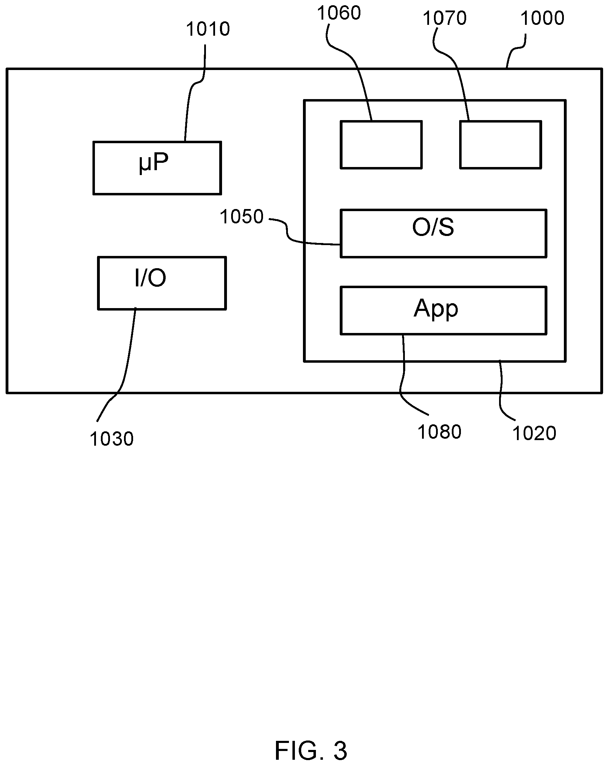

FIG. 3 illustrates an example of a computer 1000 within which one or more parts of an embodiment may be employed. Various operations discussed above may utilize the capabilities of the computer. For example, one or more parts of a system according to an embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 1000 includes, but is not limited to, PCs, workstations, laptops, PDAs, palm devices, servers, storages, microcontroller units, integrated processors, AI-accelerators, and the like. Generally, in terms of hardware architecture, the computer may include one or more processors 1010, memory, and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory. The processor 1010 can be virtually any custom made or commercially available processor, a central processing unit (CPU), a digital signal processor (DSP), a tensor processing unit (TSP) specifically designed for neural processing, a dedicated AI accelerator/processing unit or an auxiliary processor among several processors associated with the computer, and the processor may be a semiconductor based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read-only memory (EPROM), electronically erasable programmable read-only memory (EEPROM), programmable read-only memory (PROM), tape, compact disc read-only memory (CD-ROM), disk, diskette, cartridge, cassette or the like, etc.). Moreover, the memory 1020 may incorporate electronic, magnetic, optical, and/or other types of storage media. Note that the memory can have a distributed architecture, where various components are situated remote from one another, but can be accessed by the processor.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application of the computer may represent various applications, computational units, logic, functional units, processes, operations, virtual entities, and/or modules in accordance with exemplary embodiments, but the application is not meant to be a limitation.

The operating system 1050 controls the execution of other computer programs, and provides scheduling, input-output control, file and data management, memory management, and communication control and related services. It is contemplated by the inventors that the application for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler), assembler, interpreter, or the like, which may or may not be included within the memory, so as to operate properly in connection with the O/S. Furthermore, the application can be written as an object-oriented programming language, which has classes of data and methods, or a procedure programming language, which has routines, subroutines, and/or functions, for example but not limited to, C, C++, C, Pascal, BASIC, API calls, HTML, XHTML, XML, ASP scripts, JavaScript, FORTRAN, COBOL, Perl, Java, ADA, NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 330 also include components for communicating over various networks, such as the Internet or intranet.

If the computer 1000 is a PC, workstation, intelligent device or the like, the software in the memory may further include a basic input output system (BIOS) (omitted for simplicity). The BIOS is a set of essential software routines that initialize and test hardware at startup, start the O/S, and support the transfer of data among the hardware devices. The BIOS is stored in some type of read-only-memory, such as ROM, PROM, EPROM, EEPROM or the like, so that the BIOS can be executed when the computer is activated.

When the computer 1000 is in operation, the processor 1010 is configured to execute software stored within the memory 1020, to communicate data to and from the memory, and to generally control operations of the computer pursuant to the software. The application 1080 and the O/S 1050 are read, in whole or in part, by the processor 1010, perhaps buffered within the processor, and then executed.

When the application 1080 is implemented in software it should be noted that the application can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method (100) for controlling a monitoring system configured to receive medical data of a subject from a plurality of sensors, and to process the medical data to generate physiological parameter values of the subject, the method comprising:
obtaining (110), for each of the plurality of sensors, a suitability score of the respective sensor;
processing (130) the obtained suitability scores associated with each of a plurality of duplicate sensors amongst the plurality of sensors to identify a redundant sensor, wherein each of the duplicate sensors are configured to generate medical data suitable for deriving a value of a same physiological parameter; and
controlling (140) the monitoring system to modify an operating state of the redundant sensor and/or to modify a processing operation applied by the monitoring system to the medical data generated by the redundant sensor.

2. The method of claim 1, wherein the suitability score indicates at least one of an accuracy of medical data generated by the sensor, an energy consumption efficiency of the sensor, a volume of medical data generated by the sensor, or a delay associated with generation of physiological parameter values from medical data generated by the sensor.

3. The method of claim 1 or 2, wherein controlling (140) the monitoring system comprises replacing or disabling a measurement operation of the redundant sensor, replacing or disabling a transfer operation for medical data generated by the redundant sensor, and/or replacing or disabling algorithms and calculations performed to generate physiological parameter values using the medical data generated by the redundant sensor.

4. The method of any of claims 1-3, wherein the suitability score comprises a confidence score indicating an accuracy of medical data generated by the sensor, and wherein obtaining (110) the suitability score of each sensor comprises:
obtaining sensor context data comprising expected sensor quality information, historical sensor quality information, sensor placement information, sensor use information, sensor interference information, and/or perceived sensor quality information; and
determining the confidence score based on the sensor context data.

5. The method of any of claims 1-4, wherein the suitability score comprises a confidence score indicating an accuracy of medical data generated by the sensor, and wherein obtaining (110) the suitability score of each sensor comprises processing, for each of the plurality of sensors, medical data obtained from the respective sensor to determine the confidence score.

6. The method of any of claims 1-5, further comprising obtaining a clinical, system and/or user requirement indicating a target characteristic of medical data relating to a physiological parameter, and wherein identifying the redundant sensor of the plurality of sensors is further based on the obtained clinical, system and/or user requirement.

7. The method of any of claims 1-5, further comprising processing (130) the obtained suitability scores associated with each duplicate sensor of the plurality of sensors to identify a further redundant sensor.

8. The method of any of claims 1-7, further comprising identifying (120) the duplicate sensors amongst the plurality of sensors.

9. The method of claim 8, wherein identifying (120) the duplicate sensors comprises:
obtaining, for each of the plurality of sensors, a list of physiological parameters derivable from medical data generated by the respective sensor; and
comparing the list of physiological parameters of each of the plurality of sensors to identify one or more sets of duplicate sensors, each set of duplicate sensors comprising sensors configured to generate medical data suitable for deriving a value of a same physiological parameter.

10. The method of claim 8, wherein identifying (120) the duplicate sensors comprises:
comparing, for each pair of sensors of the plurality of sensors, medical data generated by each sensor; and
identifying duplicate sensors based on a result of the comparison.

11. The method of any of claims 1-10, further comprising repeating the steps of obtaining (110) the suitability scores, processing (130) the obtained suitability scores, and controlling (140) the monitoring system according to a predefined loop function.

12. The method of claim 11, wherein the steps of obtaining (110) the suitability scores, processing (130) the obtained suitability scores, and controlling (140) the monitoring system are repeated responsive to an external trigger, and optionally wherein the external trigger comprises the monitoring system receiving medical data from a new sensor, a user intervention, or a change to a clinical, system and/or user requirement.

13. The method of claim 11 or 12, wherein the steps of obtaining (110) the suitability scores, processing (130) the obtained suitability scores, and controlling (140) the monitoring system are repeated responsive to a predetermined amount of time having elapsed, and optionally wherein the predetermined amount of time is based on the same physiological parameter.

14. A computer program comprising computer program code means adapted, when said computer program is run on a computer, to implement the method of any of claims 1-14.

15. A control system (200) for a monitoring system (300) configured to receive medical data of a subject from a plurality of sensors (310), and to process the medical data to generate physiological parameter values of the subject, the control system comprising:
an assessment module (210) configured to obtain, for each of the plurality of sensors, a suitability score of the respective sensor;
a redundant check module (230) configured to process the obtained suitability scores associated with each of a plurality of duplicate sensors amongst the plurality of sensors to identify a redundant sensor, wherein each of the duplicate sensors are configured to generate medical data suitable for deriving a value of a same physiological parameter; and
a communication module (240) configured to control the monitoring system to modify an operating state of the redundant sensor and/or to modify a processing operation applied by the monitoring system to the medical data generated by the redundant sensor.
